# EUROPEAN PATENT APPLICATION

(11) **EP 3 492 072 A1**
(43) Date of publication of application: **05.06.2019**
(21) Application number: 18198841.1
(22) Date of filing: 26.08.2014
(51) Int. Cl.: A61K 9/28, A61K 31/225, A61K 9/20, A61P 17/06

(54) **PHARMACEUTICAL COMPOSITION CONTAINING DIMETHYL FUMARATE FOR ADMINISTRATION AT A LOW DAILY DOSE**

(30) Priority: 26.08.2013 EP 13181735; 26.08.2013 US 201361870096 P; 25.10.2013 EP 13190304; 25.10.2013 US 201361895740 P; 11.08.2014 EP 14180569; 11.08.2014 US 201462035898 P
(62) Divisional of application: 14755672.4
(71) Applicant: FWP IP APS, 1100 København K (DK)
(72) Inventor: Galetzka, Christin, 01187 Dresden (DE); Rundfeldt, Chris, 01640 Coswig (DE); Rupp, Roland, 51467 ergisch Gladbach (DE); Andersen, Peder M., 1561 Copenhagen V (DK)
(74) Representative: Pohlman, Sandra M.

(57) **Abstract**

The present invention relates to pharmaceutical compositions containing dimethyl fumarate (DMF). More specifically, the present invention relates to a pharmaceutical composition for oral use in treating psoriasis by administering a low daily dosage in the range of 375 mg ± 5% dimethyl fumarate, wherein the pharmaceutical formulation is in the form of an erosion matrix tablet.

## Description

### FIELD OF THE INVENTION

The present invention relates to pharmaceutical compositions containing dimethyl fumarate (DMF). More specifically, the present invention relates to a pharmaceutical composition for oral use in treating psoriasis (including moderate to severe plaque psoriasis) by administering a low daily dosage in the range of 375 mg ± 5% dimethyl fumarate, wherein the pharmaceutical formulation is in the form of an erosion matrix tablet.

### BACKGROUND OF THE INVENTION

Fumaric acid esters, i.e. dimethyl fumarate in combination with salts of ethylhydrogen fumarate have been used in the treatment of psoriasis for many years. The combination is marketed under the trade name Fumaderm®. It is in the form of enteric coated tablets for oral use.

Fumaderm® is available in two different dosage strengths (Fumaderm® intial and Fumaderm®):

| | Fumaderm® inital | Fumaderm® |
|---|---|---|
| Dimethyl fumarate Ethylhydrogen fumarate, | 30 mg | 120 mg |
| Ca-salt Ethylhydrogen fumarate, | 67 mg | 87 mg |
| Mg-salt Ethylhydrogen fumarate, | 5 mg | 5 mg |
| Zn-salt | 3 mg | 3 mg |

The two strengths are intended to be applied in an individually based dose regimen starting with Fumaderm® initial in an escalating dose, and then after e.g. three weeks of treatment switching to Fumaderm®. However, a high frequency of side effects causes some patient discontinuation early in treatment. It is contemplated that the gastrointestinal side effects and flushing can, at least partially, be explained by the release properties of the prescription formulation, leading to high local drug concentration on the intestinal mucosa and subsequent high plasma concentration of drug metabolite.

To reduce the side effects, EP-A-1 131 065, EP-A-1 059 920 and EP-A-1 123 092 suggest the preparation of DMF and/or MMF containing microtablets.

Another approach to reduce unwanted side effects is the preparation of controlled-release formulations as disclosed in WO 2006/037342 A2.

Furthermore, specific controlled-release formulations are disclosed in WO 2010/079222 A1. Such controlled-release formulations comprise one or more fumaric acid esters in an erosion matrix tablet having an enteric coating that is thinner than enteric coatings usual in the art. The pharmaceutical formulations disclosed in WO 2010/079222 show excellent pharmacokinetic parameters.

The DMF formulations and dosing regimens administered in clinical trials have been associated with gastrointestinal (GI) side-effects, such as flushing, diarrhea, stomach ache, stomach pain, abdominal pain, abdominal cramps, nausea, flatulence, tenesmus, meteorism, an increased frequency of stools, a feeling of fullness, and/or upper abdominal cramps.

In view of the above, it is the object to be solved by the present invention to lower the daily dose of dimethyl fumarate in an effective oral formulation for use in the treatment of psoriasis (including moderate to severe plaque psoriasis).

### DISCLOSURE OF THE INVENTION

It has been surprisingly found that the solution to solve the above object is to use dimethyl fumarate as the sole active ingredient in an erosion matrix tablet at a dose of 375 mg ± 5% per day.

The erosion matrix formulation will enable the slow and controlled release of the active ingredient DMF within the enteric lumen. The slow release enables the exposure of the enteral immune system, even prior to absorption into the systemic circulation and during the absorption process, to the active principle for a prolonged period of time. This local exposure induces immune modulation locally in addition to the systemic pharmacological action. These locally modulated immune cells mediate the systemic pharmacological action, in addition to possible systemic effects. By this slow and controlled release, the activity of DMF on the cells in the local immune system is improved, making a pharmacological activity of the drug at the unexpected low dose level of 375 mg ± 5% per day possible.

More specifically, the present invention concerns the following aspects:
According to the first aspect, the present invention is directed to a pharmaceutical composition for oral use in treating psoriasis, wherein said composition is in the form of an erosion matrix tablet comprising a tablet core and one or more coating(s), wherein the tablet core comprises
i) 10 to 80 % by weight dimethyl fumarate as an active substance, and
ii) 1 to 50 % by weight of one or more rate-controlling agents,
wherein at least one of the one or more coating(s) is an enteric coating applied at a level of 1.5 to 3.5 % by weight of the core, and
wherein the dose of dimethyl fumarate to be administered is 375 mg ± 5% per day.

According to another preferred aspect of the present invention, the tablet core of the pharmaceutical composition according to the above mentioned aspects comprises
i) 30 to 60 % by weight of dimethyl fumarate; and
ii) 3 to 40 % by weight of one or more rate-controlling agents.

It is preferred that the rate-controlling agent is a water-soluble polymer. It is even more preferred that the rate-controlling agent is a cellulose polymer or a cellulose derivative or a mixture thereof.

According to another preferred aspect of the present invention, the rate-controlling agent is selected from the group comprising hydroxypropyl cellulose, hydroxypropyl methyl cellulose (HMPC), methyl cellulose, carboxymethyl cellulose and mixtures thereof. Most preferably, the rate-controlling agent is hydroxypropyl cellulose.

According to another preferred aspect of the present invention, the tablet core of the pharmaceutical composition according to any one of the above aspects comprises a binder. According to a preferred embodiment, said binder is lactose.

According to more specific embodiments of the present invention, the tablet core of the pharmaceutical composition according to any one of the above aspects comprises:
i) 35 - 55 % by weight of dimethyl fumarate;
ii) 3 - 12 % by weight of hydroxypropyl cellulose; and
iii) 40 - 60 % by weight of lactose.

More preferably, said tablet core comprises:
i) 40 - 50 % by weight of dimethyl fumarate;
ii) 3 - 12 % by weight of hydroxypropyl cellulose; and
iii) 45 - 55 % by weight of lactose.

It is even more preferred that said tablet core comprises:
i) 42 - 48 % by weight of dimethyl fumarate;
ii) 3 - 10 % by weight of hydroxypropyl cellulose; and
iii) 45 - 52 % by weight of lactose.

In another aspect of the present invention, the amount of hydroxypropyl cellulose is 3 - 6 % by weight in the above defined tablet cores.

According to further preferred embodiments of the present invention, said tablet core of said pharmaceutical composition according to any one of the above aspects further comprises 0.15 - 0.7 % by weight of magnesium stearate and, optionally, 0.05 to 0.25 % by weight of silicon dioxide.

In one aspect of the present inventions, said pharmaceutical composition is for administration once, twice or three times daily.

The pharmaceutical composition according to any one of the above aspects is for use in the treatment of psoriasis (including moderate to severe plaque psoriasis).

Furthermore, the present invention relates to the following embodiments:
1. A pharmaceutical composition in the form of an erosion matrix tablet comprising a tablet core and one or more coating(s), wherein the tablet core comprises
   i) 10 to 80 % by weight dimethyl fumarate as an active substance, and
   ii) 1 to 50 % by weight of one or more rate-controlling agents,
   wherein at least one of the one or more coating(s) is an enteric coating applied at a level of 1.5 - 3.5 % by weight of the core, and
   wherein the erosion matrix tablet comprises from 375 mg ± 5% of dimethyl fumarate.
2. The pharmaceutical composition of item 1 above, wherein the tablet core comprises
   i) 30 to 60 % by weight of dimethyl fumarate; and
   ii) 3 to 40 % by weight of one or more rate-controlling agents.
3. The pharmaceutical composition of item 1 or 2, wherein the rate-controlling agent is a water-soluble polymer.
4. The pharmaceutical composition of any one of items 1-3, wherein the rate-controlling agent is a cellulose polymer or a cellulose derivative or a mixture thereof.
5. The pharmaceutical composition of item 4, wherein the rate-controlling agent is selected from the group comprising hydroxypropyl cellulose, hydroxypropyl methyl cellulose (HPMC), methyl cellulose, carboxymethyl cellulose and mixtures thereof.
6. The pharmaceutical composition of item 5, wherein the rate-controlling agent is hydroxypropyl cellulose.
7. The pharmaceutical composition of any one of the preceding items, wherein the tablet core further comprises a binder.
8. The pharmaceutical composition of item 7, wherein the binder is lactose.
9. The pharmaceutical composition of any one of the preceding items, wherein the tablet core comprises:
   i) 35 - 55 % by weight of dimethyl fumarate;
   ii) 3 - 12 % by weight of hydroxypropyl cellulose; and
   iii) 40 - 60 % by weight of lactose.
10. The pharmaceutical composition of item 9, wherein the tablet core comprises:
   i) 40 - 50 % by weight of dimethyl fumarate;
   ii) 3 - 12 % by weight of hydroxypropyl cellulose; and
   iii) 45 - 55 % by weight of lactose.
11. The pharmaceutical composition of item 10, wherein the tablet core comprises:
   i) 42 - 48 % by weight of dimethyl fumarate;
   ii) 3 -10 % by weight of hydroxypropyl cellulose; and
   iii) 45 - 52 % by weight of lactose.
12. The pharmaceutical composition of any one of the preceding items, wherein the tablet core comprises:
   i) 35 - 55 % by weight of dimethyl fumarate;
   ii) 3 - 6 % by weight of hydroxypropyl cellulose; and
   iii) 40 - 60 % by weight of lactose.
13. The pharmaceutical composition of item 12, wherein the tablet core comprises:
   i) 40 - 50 % by weight of dimethyl fumarate;
   ii) 3 - 6 % by weight of hydroxypropyl cellulose; and
   iii) 45 - 55 % by weight of lactose.
14. The pharmaceutical composition of item 13, wherein the tablet core comprises:
   i) 42 - 48 % by weight of dimethyl fumarate;
   ii) 3 - 5.5 % by weight of hydroxypropyl cellulose; and
   iii) 45 - 52 % by weight of lactose.
15. The pharmaceutical composition of any one of the preceding items, wherein the tablet core further comprises 0.15 - 0.7 % by weight of magnesium stearate and, optionally, 0.05 to 0.25 % by weight of silicon dioxide.
16. The pharmaceutical composition of any one of the preceding items, wherein the erosion matrix tablet comprises about 375 mg of dimethyl fumarate.
17. The pharmaceutical composition of any one of the preceding items, wherein the dimethylfumarate is in the form of a crystalline powder.
18. A method of treating psoriasis in a subject in need thereof, said method comprising administering to said subject a pharmaceutical formulation in the form of an erosion matrix tablet comprising a tablet core and one or more coating(s), wherein the tablet core comprises
   i) 10 to 80 % by weight dimethyl fumarate as an active substance, and
   ii) 1 to 50 % by weight of one or more rate-controlling agents,
   wherein at least one of the one or more coating(s) is an enteric coating applied at a level of 1.5 - 3.5 % by weight of the core, and
   wherein the dose of dimethyl fumarate to be administered is from 375 mg ±5% per day.
19. The method of item 18, wherein the tablet core comprises
   i) 30 to 60 % by weight of dimethyl fumarate; and
   ii) 3 to 40 % by weight of one or more rate-controlling agents.
20. The method of item 18 or 19, wherein the rate-controlling agent is a water-soluble polymer.
21. The method of any one of items 18-29, wherein the rate-controlling agent is a cellulose polymer or a cellulose derivative or a mixture thereof.
22. The method of item 21, wherein the rate-controlling agent is selected from the group comprising hydroxypropyl cellulose, hydroxypropyl methyl cellulose (HPMC), methyl cellulose, carboxymethyl cellulose and mixtures thereof.
23. The method of item 22, wherein the rate-controlling agent is hydroxypropyl cellulose.
24. The method of any one of items 18-23, wherein the tablet core further comprises a binder.
25. The method of items 24, wherein the binder is lactose.
26. The pharmaceutical composition of any one of items 18-25, wherein the tablet core comprises:
   i) 35 - 55 % by weight of dimethyl fumarate;
   ii) 3 - 12 % by weight of hydroxypropyl cellulose; and
   iii) 40 - 60 % by weight of lactose.
27. The pharmaceutical composition of item 26, wherein the tablet core comprises:
   i) 40 - 50 % by weight of dimethyl fumarate;
   ii) 3 - 12 % by weight of hydroxypropyl cellulose; and
   iii) 45 - 55 % by weight of lactose.
28. The pharmaceutical composition of item 27, wherein the tablet core comprises:
   i) 42 - 48 % by weight of dimethyl fumarate;
   ii) 3 -10 % by weight of hydroxypropyl cellulose; and
   iii) 45 - 52 % by weight of lactose.
29. The method of any one of items 18-25, wherein the tablet core comprises:
   i) 35 - 55 % by weight of dimethyl fumarate;
   ii) 3 - 6 % by weight of hydroxypropyl cellulose; and
   iii) 40 - 60 % by weight of lactose.
30. The method of item 29, wherein the tablet core comprises:
   i) 40 - 50 % by weight of dimethyl fumarate;
   ii) 3 - 6 % by weight of hydroxypropyl cellulose; and
   iii) 45 - 55 % by weight of lactose.
31. The method of item 30, wherein the tablet core comprises:
   i) 42 - 48 % by weight of dimethyl fumarate;
   ii) 3 - 5.5 % by weight of hydroxypropyl cellulose; and
   iii) 45 - 52 % by weight of lactose.
32. The method of any one of items 18-31, wherein the tablet core further comprises 0.15 - 0.7 % by weight of magnesium stearate and, optionally, 0.05 to 0.25 % by weight of silicon dioxide.
33. The method of any one of items 18-32, wherein the dose of dimethyl fumarate to be administered is about 375 mg of dimethyl fumarate per day.
34. The method of any one of items 18-27, wherein the dimethylfumarate is in the form a crystalline powder.
35. The method of any one of items 18-34, wherein the composition is administered once, twice or three times daily.
36. The method of item 35, wherein the erosion matrix tablet comprises from 375 mg ±5% of dimethyl fumarate, and the erosion matrix tablet is administered once daily.
37. The method of item 36, wherein the erosion matrix tablet comprises about 375 mg of dimethyl fumarate.
38. The method of item 18, wherein said method comprises administering to the subject in need thereof the erosion matrix tablet of item 1 above, wherein following oral administration under fasting conditions of the erosion matrix tablet monomethylfumarate appears in the plasma of the subject upon hydrolysis of dimethylfumarate and the Cmax of the monomethylfumarate in the plasma of the subject is between about 0.3 mg/L and about 2 mg/L.
39. The method of item 18, wherein said method comprises administering to the subject in need thereof the erosion matrix tablet of item 1, wherein following oral administration under fasting conditions of the erosion matrix tablet monomethylfumarate appears in the plasma of the subject upon hydrolysis of dimethylfumarate and the Tmax of the monomethylfumarate in the plasma of the subject is between about 1.5 h and about 4.5 h.
40. The method of item 18, wherein said method comprises administering to the subject in need thereof the erosion matrix tablet of item 1, wherein following oral administration under fasting conditions of the erosion matrix tablet monomethylfumarate appears in the plasma of the subject upon hydrolysis of dimethylfumarate and the circulating plasma concentration of the monomethylfumarate in the plasma of the subject starts within the first hour after administration, has at least 50% of the achieved Cmax over about 1 h to 4.5 h and can be measured in total over the course of about 5 h to 8 h.
41. The method of item 40, wherein the subject in need thereof has mild to moderate plaque psoriasis, moderate to severe plaque psoriasis, or severe plaque psoriasis.

### DETAILED DESCRIPTION OF THE PRESENT INVENTION

The pharmaceutical composition in the form of a matrix tablet according to the present application is a controlled-release formulation that releases the active ingredient, i.e. dimethyl fumarate, in a sustained manner. More specifically, the erosion matrix preferably results in release of the dimethyl fumarate - when subjected to an *in vitro* dissolution test carried out at 37°C and a paddle speed of 100 rpm employing 0.1 N hydrochloric acid as dissolution medium during the first 2 hours of the test and then 0.05 M phosphate buffer pH 6.8 as dissolution medium - as follows:
within the first 2 hours after start of the test from about 0% w/w to about 10 % w/w, preferably 0% w/w to about 5 % w/w, more preferably 0 % w/w to < 2 % w/w of the total amount of dimethyl fumarate contained in the pharmaceutical composition is released, and
within the first 2.5 hours after start of the test from about 2% w/w to about 20% w/w of the total amount of the dimethyl fumarate contained in the pharmaceutical composition is released, and
within the first 3.5 hours after start of the test from about 35 % w/w to about 65 % of the total amount of dimethyl fumarate contained in the pharmaceutical composition is released, and
within the first 6 hours after start of the test > 85 % w/w of the total amount of dimethyl fumarate contained in the pharmaceutical composition is released.

In the present context the term "API", which is an abbreviation for "active pharmaceutical ingredient" and the term "active substance" are used interchangeably and refers to dimethyl fumarate that is to be released from the pharmaceutical formulation according to the invention.

With respect to *in vitro* methods, well-established methods are available, especially methods described by official monographs like *e.g.* United States Pharmacopeia (USP) or the European Pharmacopoeia. A person skilled in the art will know which method to choose and how to select the specific conditions to carry out the *in vitro* test. For instance, the USP prescribes *in vitro* tests be carried out at 37 +/- 1.0 such as 37 +/-0.5 degrees Celsius/Centigrade. In one aspect, a suitable dissolution test is one, wherein the dissolution profile is determined as described in the United States Pharmacopoeia at 37°C using a paddle dissolution apparatus at 100 rpm employing 0.1 N hydrochloric acid as dissolution medium during the first 2 hours of the test and then followed by 0.05 M phosphate buffer pH 6.8 as dissolution medium for the remaining test period. A person skilled in the art will know how to adjust the conditions applied, e.g. temperature, pH, paddle speed, duration etc. In a further aspect, the *in vitro* dissolution testing is carried out as follows: A USP apparatus II (paddles) with 1 litre vessels is used. Bath temperature is set to 37°C±0.5°C and paddle speed to 100 rpm. One tablet is placed in one vessel containing 750 ml 0.1N HCI (pH 1.2) over 2 h. After that the pH is changed to 6.8 by adding 220 ml -250 ml 0.2 M sodium phosphate buffer. 2.5 ml samples are taken at least after 2 h, 2.5 h, 3.5 h and 6 h, immediately stored at 2-8°C and analyzed by HPLC for DMF. The HPLC parameters are set as follows: Column: Phenomenex Luna C18, 50 x 4.6 mm, 3 µm; column oven temperature 30°C, :mobile phase: Methanol:20 mM phosphate buffer pH 3.0 (35:65 V/V), inject volume: 5 µl, Flow rate: 0.8 ml/min, UV-Detector wavelength: 210 nm, run time 5 min, DMF retention time 3.5 min.

The release *in vivo* may be tested by measuring the plasma concentration at predetermined time periods and thereby obtaining a plasma concentration versus time profile for the dimethyl fumarate or, if relevant, a metabolite thereof. Furthermore, it is contemplated that metabolism already takes place within the gastro-intestinal tract or during passage of the gastro-intestinal mucosa. Accordingly, when dimethyl fumarate is administered, the relevant component to search for in the plasma may be the monomethyl ester and not the dimethylester of fumaric acid.

Other tests may also be used to determine or to give a measure of the release of the active substance *in vivo.* Thus, animals (*e.g.* minipigs, dogs, monkeys etc.) may be used as a model. The animals receive the compositions under investigation and after specified periods of time, blood samples are collected and the content of the active ingredient (or metabolite thereof, if relevant) is determined in plasma or specific organs or extracted from the intestinal contents.

Another test involves the use of a specific segment of an animal or human intestine. The segment is placed in a suitable apparatus containing two compartments (a donor and a receiver) separated by the segment, and the composition under investigation is placed in a suitable medium in one compartment (the donor compartment). The composition will release the active substance that subsequently is transported across the intestinal segment. Accordingly, at suitable time intervals, the concentration of the active substance (or, if relevant, the metabolite) is measured in the receiver compartment.

A person skilled in the art will be able to adapt the above-mentioned method to the specific composition.

In the present context, the term "relative bioavailability" refers to a comparison of the amount of drug absorbed in vivo (expressed as area under the curve (AUC)) after administration of two different formulations or reference product. In the present context, the amount of drug absorbed, expressed as AUC, can be detected in the form of the actual drug administered, or as a metabolite thereof. The relative bioavailability can be expressed as a percentage of a reference AUC, i.e. AUC %.

In the present context the term "variability" refers to the variability of PK parameters (e.g. Cₘₐₓ and AUC) after administration of a pharmaceutical formulation or a reference formulation. The variability can be expressed as the coefficient of variation (CV) for a PK parameter, i.e. the ratio of the standard deviation to the mean. Reference to PK parameter values herein, such as Cmax and Tmax, refers to mean values obtained from human clinical studies in either fed or fasted subjects.

In the present context the term "tolerability" refers to the potential of a drug to be endured by subjects and/or patients. In one aspect, "tolerability" is determined as the potential of a drug to be endured by subjects and/or patients in early stages of treatment, such as within the first three months of start of therapy, such as within the first month of start of therapy, such as within the first two weeks of start of therapy, such as within the first week of start of therapy, such as within the first three days of start of therapy, such as within the first day of start of therapy, such as after the first dose of the therapy. A drug with better tolerability produces fewer side effects in a subject and/or patient c.f. a drug with worse tolerability.

In the present context the term "substantial absence of" refers to a level of less than about 1 %, such as less than about 0.5 %, such as less than about 0.3 %, such as about 0.0 %.

In the present context the terms "rate-controlling agent" and "rate-controlling agent in the form of a polymeric matrix material" are used interchangeably and refer to an agent that is able to sustain and/or prolong the *in vivo* and/or *in vitro* release of the active substance.

As mentioned above, the *in vivo* and/or *in vitro* release of the active substance is "controlled", i.e. prolonged and/or slow compared with the commercially available Fumaderm® composition. In the present context, the term "controlled" is intended to indicate that the active substance is released during a longer time period than Fumaderm® such as at least during a time period that is at least 1.2 times, such as, e.g., at least 1.5 times, at least 2 times, at least 3 times, at least 4 times or at least 5 times greater than that of Fumaderm®. Thus, if e.g. 100% of dimethyl fumarate is released from Fumaderm® tablets 3 hours after the start of a suitable test, then 100% of dimethyl fumarate in a composition according to the invention is released at least 3.6 hours after the start of a suitable test.

The formulation according to the invention is contemplated to provide improved tolerability, such as fewer and/or less severe gastrointestinal (GI) side-effects, such as fewer and/or less severe redness episodes, such as fewer and/or less severe flushing episodes.

As used in the present invention, a gastrointestinal (GI) side effect may include, but is not limited to diarrhea, emesis, stomach ache, stomach pain, abdominal pain, abdominal cramps, nausea, flatulence, tenesmus, meteorism, an increased frequency of stools, a feeling of fullness and upper abdominal cramps.

In the present context, a reduction of GI related side effects is intended to denote a decrease in severity and/or incidence among a given treated patient population, comparing the GI side effects observed after administration of the formulation according to the invention to the GI side effects observed after administration of Fumaderm®. A reduction in GI related side effects according to this definition could thus be construed as a substantial reduction in incidence of any of the GI side effect listed above, such as at least a 10% reduction in incidence or more preferably at least 20 % reduction in incidence or even more preferable a more than 30 % reduction in incidence. A reduction in GI related side effect can also be expressed as a substantial reduction in severity in any of the GI side effects listed above, such as a reduction in severity and/or frequency of diarrhea, emesis, stomach ache, stomach pain, abdominal pain, abdominal cramps, nausea, flatulence, tenesmus, meteorism, increased frequency of stools, a feeling of fullness or upper abdominal cramps. The reduction of GI related side effects, as described above, can be monitored in a clinical trial setting, either comparing the administration of the formulation according to the invention head on with Fumaderm® or with placebo. In case of a placebo controlled trial, the incidence of GI related side effects in the patients receiving the formulation according to the invention compared to the placebo group, can be compared to historical trials comparing Fumaderm® to placebo (see e.g. Altmeyer eta/, J. Am. Acad. Dermatol. 1994; full reference: Altmeyer PJ et al.,Antipsoriatic effect of fumaric acid derivatives. Results of a multicenter double-blind study in 100 patients. J. Am. Acad. Dermatol. 1994; 30:977-81).

In a further aspect, the formulation according to the invention - upon oral administration and in comparison to that obtained after oral administration of Fumaderm® tablets in an equivalent dosage - reduce (GI) side-effects (frequency and/or severity).

WO 2010/079222 A1 discloses that an erosion matrix formulation upon oral administration and in comparison to that obtained after oral administration of Fumaderm® tablets in an equivalent dosage reduces unwanted side effects, in particular flushing (frequency and/or severity). Frequency and strength of these and other side effects are further reduced in view of the low daily dosage of 375 mg ± 5 %.

In the present context the term "flushing" describes episodic attacks of redness of the skin together with a sensation of warmth or burning of the face and/or neck, and less frequently the upper trunk and abdomen or the whole body. It is the transient nature of the attacks that distinguishes flushing from the persistent erythema of photosensitivity or acute contact reactions. Repeated flushing over a prolonged period of time can lead to telangiectasia and occasionally to classical rosacea of the face (Greaves MW. Flushing and flushing syndromes, rosacea and perioral dermatitis. In: Champion RH, et al, eds. Rook/Wilkinson/Ebling textbook of dermatology, 6th ed., vol. 3. Oxford, UK: Blackwell Scientific, 1998: 2099-2104).

In the present context, a reduction of flushing is intended to denote a decrease in severity and/or incidence/frequency among a given treated patient population of flushing observed after administration of the formulation according to the invention compared with flushing observed after administration of Fumaderm® and can be measured e.g. as described by O'Toole et al. Cancer 2000, 88(4): p. 770-776. A reduction in flushing according to this definition could thus be construed as a reduction in incidence and/or severity of flushing. In one aspect of the invention, the incidence of flushing is reduced by at least about a quarter, in another aspect of the invention the incidence is reduced by at least about a third, in another aspect of the invention the incidence is reduced by at least about half, and in a further aspect of the invention, the flushing incidence is reduced by about two thirds or more. Likewise, the severity is in one aspect of the invention reduced by at least about a quarter, in another aspect of the invention by at least about a third, in another aspect of the invention by at least half, and in a further aspect of the invention by at least about two thirds. A one hundred percent reduction in flushing incidence and severity is most preferable, but is not required. The reduction of flushing, as described above, can be monitored in a clinical trial setting, e.g. comparing the administration of the compound according to the invention with e.g. administration of Fumaderm®. In case of a Fumaderm® controlled trial, the incidence and severity, defined as mild, moderate or severe, of flushing in the patients receiving the compound according to the invention compared to the Fumaderm® group, can be compared.

In one aspect, the severity of flushing is determined as the body surface area involved.
In one embodiment, such a clinical trial can be carried out as described above under "Clinical trial in patients". In another embodiment, such a clinical trial can be carried out as described above under "Clinical trial in healthy volunteers".

In a further aspect, the formulation according to the invention - upon oral administration and in comparison to that obtained after oral administration of Fumaderm® tablets in an equivalent dosage - reduce redness (frequency and/or severity).

In the present context the term "redness" describes episodic attacks of redness of the skin. In one aspect, the redness occurs in the face, neck, and less frequently the upper trunk and abdomen.

In the present context, a reduction of redness is intended to denote a decrease in severity and/or incidence/frequency among a given treated patient population of redness observed after administration of the formulation according to the invention compared with redness observed after administration of Fumaderm® and can e.g. be assessed by a clinician or nurse.

A reduction in redness according to this definition could thus be construed as a reduction in incidence and/or severity of redness. In one aspect of the invention, the incidence of redness is reduced by at least about a quarter, in another aspect of the invention the incidence is reduced by at least about a third, in another aspect of the invention the incidence is reduced by at least about half, and in a further aspect of the invention, the redness incidence is reduced by about two thirds or more. Likewise, the severity is in one aspect of the invention reduced by at least about a quarter, in another aspect of the invention by at least about a third, in another aspect of the invention by at least half, and in a further aspect of the invention by at least about two thirds. A one hundred percent reduction in redness incidence and severity is most preferable, but is not required. The reduction of redness, as described above, can be monitored in a clinical trial setting, e.g. comparing the administration of the compound according to the invention with e.g. administration of Fumaderm®. In case of a Fumaderm® controlled trial, the incidence and severity, defined as mild, moderate or severe, of redness in the patients receiving the compound according to the invention compared to the Fumaderm® group, can be compared.

In one aspect, the severity of redness is determined as the body surface area involved.

In one embodiment, such a clinical trial can be carried out as described above under "Clinical trial in patients".

In another embodiment, such a clinical trial can be carried out as described above under "Clinical trial in healthy volunteers".

In one embodiment, the relative bioavailability of the formulation of the invention compared to Fumaderm® is at least about 75%, such as at least about 80%, such as at least about 85%, such as at least about 90%, such as at least about 95%, such as about 100%.

In the present context the term "erosion matrix" refers to a matrix wherein the release of the API does not depend upon intrinsic diffusion processes but rather is the result of the rate of the matrix erosion. By stripping off the erodible matrix layers in a well-controlled manner, predetermined amounts of the API will be obtained, with the release of API being dependent on the rate of swelling and dissolution or erosion of the matrix and on the rate of dissolution, solubility and rate of diffusion of the API.

In an aspect of the invention, the rate-controlling agent is a water-soluble polymer. As used herein, the term "water-soluble polymer" means a conventional polymer for pharmaceutical use, having a solubility of more than 10 mg/ml in water. Suitable water- soluble polymers includes, but are not limited too, for example, hydroxypropylmethyl cellulose, hydroxypropyl cellulose, methyl cellulose and carboxymethyl cellulose. According to a preferred embodiment, the water-soluble polymer is hydroxypropyl cellulose.

As used herein, the term "water-insoluble polymer" means a conventional polymer for pharmaceutical use, having a solubility of not more than 10 mg/ml in water.

In a further aspect of the invention, the erosion matrix contains essentially no water-insoluble polymer. In yet a further aspect, the erosion matrix contains no water-insoluble polymer.

In the present context the term "essentially no" refers to a level of less than about 1 %, such as less than about 0.5 %, such as less than about 0.3 %, such as about 0.0 %.

In an aspect of the invention, the rate-controlling agent is a water-soluble polymer and the erosion matrix contains essentially no water-insoluble polymer.

According to a preferred aspect of the invention, the rate-controlling agent is a water-soluble polymer and the erosion matrix contains no water-insoluble polymer.

In an embodiment of the invention, the rate-controlling agent is a cellulose polymer or a cellulose derivative or a mixture thereof. As non-limiting examples of a cellulose polymer or a cellulose derivative or a mixture thereof may be mentioned hydroxypropyl cellulose, hydroxypropyl methyl cellulose (HPMC), methyl cellulose, carboxymethyl cellulose and mixtures thereof.

According to the most preferred embodiment of the present invention, the rate-controlling agent is hydroxypropyl cellulose.

Many different grades of hydroxypropyl cellulose exist depending on e.g. the molecular weight thereof, the degree of etherification, viscosity etc. Non-limiting exemplary embodiments of commercially available hydroxypropyl celluloses are obtainable from e.g. 5 Aqualon or Nippon Soda under the trade names Klucel® HPC-L, HPC-SL, HPC-SSL, HPC-M, HPC-H etc. In an embodiment of the invention, the rate-controlling agent is hydroxypropyl cellulose having a viscosity (mPa.s) of 3.0-5.9 as measured in an aqueous solution containing 2% by weight of dry HPC at 20°C. In an embodiment of the invention, the rate-controlling agent is HPC-SL.

According to the present invention, the rate-controlling agent is present in an amount of 1-50 % by weight, 1- 40 % by weight, such as 3 - 35 % by weight, such as 4-15 % by weight, such as 4-10 % by weight, such as 3-15 % by weight, , such as 3-12 % by weight, such as 3-10 % by weight, such as 3-6 % by weight, such as 3-5.5 % by weight, and such as 4-6 % by weight.

In an aspect, the present invention relates to a pharmaceutical formulation comprising an erosion matrix which comprises:
i) 10 % to 80 %, such as 20 % to 70 %, such as 20 % to 60 %, such as 30 % to 60 %, such as 35 % to 60 %, such as 35 % to 55 %, such as 40 % to 55 %, such as 40 % to 50 %, such as 44 % to 55 %, such as 42 % to 48 %, by weight of dimethyl fumarate as an active substance; and
ii) 1 % to 50 %, such as 1 % to 40 %, such as 3 % to 40 %, such as 3 % to 20 % by weight of one or more rate-controlling agents;
wherein erosion of said erosion matrix permits controlled release of said active substance.

The amount of rate-controlling agent varies in accordance with the specific rate-controlling agent used, the release profile aimed at, the level and nature of any excipients and additives present in the core tablet, etc.

According to a preferred embodiment of the invention, the pharmaceutical composition further comprises a binder.

Non-limiting examples of a binder include water-soluble sugars and sugar alcohols, such as lactose, saccharose, glucose, sorbitol, mannitol etc. In a particularly preferred embodiment, said binder is lactose. Lactose is commercially available in a number of different grades depending i.e. on the manufacturing method used resulting in a range of particle sizes, particle size distributions etc. Examples of lactose include, but are not limited to anhydrous lactose, lactose made from alpha-lactose-monohydrate, agglomerated lactose, granulated lactose, crystalline lactose, crystalline, sieved lactose, sieved lactose (e.g. PrismaLac®, such as PrismaLac® 40), crystalline, abrasive lactose (e.g. GranuLac®, such as GranuLac® 70, GranuLac® 140, GranuLac® 200, GranuLac® 230 and GranuLac® 400), improved lactose, agglomerated lactose (e.g. Tablettose®, such as Tablettose® 70, Tablettose® 80 and Tablettose® 100), improved lactose, spraydried lactose (FlowLac®, such as FlowLac® 90 and FlowLac® 100). Lactose is available from e.g. Meggle Pharma under the trade names PrismaLac®, Capsulac®, such as Capsulac®60, SacheLac®, SpheroLac®, Inhalac® GranuLac®, such as GranuLac® 70, GranuLac® 140, GranuLac® 200, GranuLac® 230 and GranuLac® 400, SorboLac®, Tablettose®, such as Tablettose® 70, Tablettose® 80 and Tablettose® 100, 25 FlowLac®, such as FlowLac® 90 and FlowLac® 100.

In one aspect, the lactose is agglomerated lactose. In another aspect, the lactose is spraydried lactose. In another aspect, the lactose is abrasive lactose.

In an embodiment of the invention, the tablet core of the pharmaceutical composition according to the invention comprises:
i) 40 to 60 % by weight of dimethyl fumarate as an active substance; and
ii) 40 to 60 % by weight, preferably, 45 to 55 % by weight, of a binder, preferably lactose.

According to a preferred embodiment of the present invention, the tablet core of the pharmaceutical composition according to the present invention comprises:
i) 35 % to 55 % by weight dimethyl fumarate as an active substance;
ii) 3 to 12 % by weight of a rate-controlling agent;
iii) 40 to 60 % by weight of a binder.

Even more preferred is an embodiment, wherein the tablet core of the pharmaceutical composition of the present invention comprises:
i) 40 to 50 % by weight of dimethyl fumarate as an active substance;
ii) 3 to 12 % by weight of hydroxypropyl cellulose, in particular 3 to 6 % by weight;
iii) 45 to 55 % by weight of lactose.

In another preferred embodiment of the invention, the tablet core of the pharmaceutical composition according to the present invention comprises:
i) 42 % to 48 % by weight of dimethyl fumarate;
ii) 3 to 10 % by weight of hydroxypropyl cellulose, in particular 3 to 6 % by weight;
iii) 45-52 % by weight of lactose.

In an embodiment the composition according to the invention further comprises one or more lubricants.

In a preferred embodiment the pharmaceutical composition according to the invention further comprises one or more lubricant(s) and/or one or more flow control agent(s).

More specifically, in an embodiment of the invention, the tablet core of the pharmaceutical composition according to the present invention comprises:
i) 35 to 55 % by weight of dimethyl fumarate as an active substance;
ii) 3 to 12 % by weight of rate-controlling agent, such as hydroxylpropyl cellulose;
iii) 40 to 60 % by weight of binder, such as lactose;
iv) 0.15 and 0.7 % by weight of lubricant, such as magnesium stearate;
and optionally 0.05-0.25 % by weight of flow control agents, such as silicon dioxide.

In an even more preferred embodiment of the invention, the tablet core of the pharmaceutical composition according to the invention comprises:
i) 40 to 50 % by weight of dimethyl fumarate as an active substance;
ii) 3 to 12 % by weight of rate-controlling agent, such as hydroxylpropyl cellulose;
iii) 45 to 55 % by weight of binder, such as lactose;
iv) 0.15-0.7 % by weight of lubricant, such as magnesium stearate;
and optionally 0.05-0.25 % by weight of flow control agents, such as silicon dioxide.

In an even more preferred embodiment of the invention, the tablet core of the pharmaceutical composition according to the invention comprises:
i) 42 to 48 % by weight of dimethyl fumarate as an active substance;
ii) 3 to 10 % by weight of rate-controlling agent, such as hydroxylpropyl cellulose;;
v) 45 to 52 % by weight of binder, such as lactose;
vi) 0.15-0.7 % by weight of lubricant, such as magnesium stearate;
and optionally 0.05-0.25 % by weight of flow control agents, such as silicon dioxide.

In another preferred embodiment of the invention, the tablet core of the pharmaceutical composition according to the invention comprises:
i) 35 to 55 % by weight of dimethyl fumarate as an active substance;
ii) 3 to 6 % by weight of rate-controlling agent, such as hydroxypropyl cellulose;
iii) 40-60 % by weight of binder, such as lactose;
iv) 0.2-0.5 % by weight of lubricant, such as magnesium stearate;
v) and optionally 0.05-0.2 % by weight of flow control agents, such as silicon dioxide.

In another even more preferred embodiment of the invention, the tablet core of the pharmaceutical composition according to the invention comprises:
i) 35 to 55 % by weight of dimethyl fumarate as an active substance;
ii) 3 to 6 % by weight of rate-controlling agent, such as hydroxypropyl cellulose;
iii) 45-55 % by weight of binder, such as lactose;
iv) 0.2-0.5 % by weight of lubricant, such as magnesium stearate;
v) and optionally 0.05-0.2 % by weight of flow control agents, such as silicon dioxide.

In the most preferred embodiment of the invention, the tablet core of the pharmaceutical composition according to the invention comprises:
i) 35 to 55 % by weight of dimethyl fumarate as an active substance;
ii) 3 to 5.5 % by weight of rate-controlling agent, such as hydroxylpropyl cellulose;
iii) 45 to 52 % by weight of binder, such as lactose;
iv) 0.2-0.5 % by weight of lubricant, such as magnesium stearate;
v) and optionally 0.05-0.2 % by weight of flow control agents, such as silicon dioxide.

According to the present invention, any pharmaceutically acceptable lubricant common in the art may be used in the pharmaceutical composition of the present invention. Magnesium stearate may be preferably used as a lubricant.

According to the present invention, any pharmaceutically acceptable flow control agent common in the art may be used in the pharmaceutical composition of the present invention. Silicon dioxide may be preferably used as a flow control agent.

In an embodiment the formulation according to the invention may further comprise pharmaceutically acceptable excipients and additives selected from the group comprising lubricants, glidants, disintegrants, flow control agents, solubilizers, pH control agents, surfactants and emulsifiers. The amounts of such excipients and additives may be adjusted such that the properties of the pharmaceutical composition are not deteriorated.

In an embodiment, the formulation according to the invention is manufactured without the use of a disintegrant, i.e. it is preferred that the pharmaceutical composition of the present invention does not contain any disintegrant. However, small amounts of a disintegrant are allowed as long as the presence of the disintegrant does not causes the erosion matrix tablet to disintegrate.

According to the present invention, at least one of the one or more coating(s) is an enteric coating.

Enteric coating materials may be selected from any of a number of commercially available 30 coating materials. Non-limiting examples thereof include Eudragit® E, L, S, L30 D-55, Kollicoat® 30D, Cellulose Acetate Phthalate, Polyvinyl Acetate Phthalate, and Hypromellose Phthalate. According to a preferred embodiment, the solution used as coating solution for preparing the essential enteric coating comprises Eudragit® L30 D-55, triethyl citrate, glycerol monostearate, and Polysorbate 80.

According to the present invention, said essential enteric coating is applied at a level of 1.5 to 3.5% by weight of the tablet core, such as 2.0 to 3.5% by weight of the tablet core, such as 2 to 3% by weight of the tablet core. According to a particularly preferred embodiment, the coating additionally fulfills the proviso that it is typically applied to a level of about 2.0 mg/cm² to about 3.5 mg/cm² of the core tablet, such as about 2.5 mg/cm² to about 3.5 mg/cm² of the core tablet, such as about 2.8 mg/cm² to about 3.3 mg/cm² of the core tablet.

Enteric coating is a well-established approach to prevent or minimise drug release in the stomach and allow release in the small intestine. Such enteric polymer coatings work on the principle of pH dependent solubility: insoluble in the low pH conditions of the stomach but soluble in the near neutral pH environment of the proximal small intestine having a pH in the range 5-6.

For drugs requiring absorption in the small intestine this leaves open only a narrow window of release, such as about 6 h, such as about 5 hours, such as about 4 hours, such as about 3 hours, hours between dissolution of the enteric coating and release of the API from the formulation. It has been found that rapid dissolution of the enteric coating is possible by the application of a relatively thin coat, i.e. an amount of 1.5 to 3.5 % by weight of enteric coating relative to the tablet core, while surprisingly still obtaining the required protection against the acid environment of the stomach as e.g. shown - when subjected to an *in vitro* dissolution test employing 0.1 N hydrochloric acid as dissolution medium during 2 hours- by less than 10%, such as less than 5%, such as less than 2%, such as about 0% release of the fumaric ester contained in the formulation.

In an embodiment of the invention, the formulation according to the invention comprises an enteric coating and the *in vivo* release of the dimethyl fumarate displays an earlier onset of release than the prior art formulation Fumaderm®, such as at least 20 minutes, at least 30 minutes, at least 40 minutes, at least 50 minutes, at least 60 minutes, at least 70 minutes, at least 80 minutes, at least 90 minutes, at least 100 minutes, at least 110 minutes, or at least 120 minutes earlier than Fumaderm® under fasting conditions.

In an embodiment of the invention, the formulation according to the invention comprises an enteric coating and the *in vivo* release of the dimethyl fumarate displays a lag time of 15 minutes to 2 hours under fasting conditions, such as a lag time of at the most 120 minutes, at the most 110 minutes, at the most 100 minutes, at the most 90 minutes, at the most 80 minutes, at the most 70 minutes, at the most 60 minutes, at the most 50 minutes, at the most 40 minutes, at the most 30 minutes, at the most 20 minutes, or at the most 15 minutes under fasting conditions.

According to a preferred embodiment of the invention, the release of the dimethyl fumarate - when subjected to an *in vitro* dissolution test employing 0.1 N hydrochloric acid as dissolution medium during the first 2 hours of the test and then 0.05 M phosphate buffer pH 6.8 as dissolution medium is as follows:
within the first 2 hours after start of the test from about 0% w/w to about 10 % w/w, preferably 0% w/w to about 5 % w/w, more preferably 0 % w/w to < 2 % w/w of the total amount of dimethyl fumarate contained in the pharmaceutical composition is released, and
within the first 2.5 hours after start of the test from about 2% w/w to about 20% w/w of the total amount of the dimethyl fumarate contained in the pharmaceutical composition is released, and
within the first 3.5 hours after start of the test from about 35 % w/w to about 65 % of the total amount of dimethyl fumarate contained in the pharmaceutical composition is released, and
within the first 5 hours after start of the test > 85 % w/w of the total amount of dimethyl fumarate contained in the pharmaceutical composition is release.

In an embodiment the pharmaceutical composition according to the invention is for administration once, twice or three times daily.

According to a preferred embodiment, the pharmaceutical composition is for administration once daily. In such a case, the pharmaceutical composition according to the present invention may preferably contain a total amount of dimethyl fumarate as the active substance of 375 mg ± 5%, preferably about 375 mg.

According to another preferred embodiment according to the present invention, the pharmaceutical composition is for administration three times daily. In such a case the pharmaceutical composition preferably contains a total amount of dimethyl fumarate of 125 mg.

The daily dosage of the controlled release pharmaceutical composition according to the invention that is administered to treat a patient depends on a number of factors among which are included, without limitation, weight and age and the underlying causes of the condition or disease to be treated, and is within the skill of a physician to determine.

According to the present invention, the daily dosage of dimethyl fumarate is 375 mg ± 5%, i.e. a range of 356.25 to 393.75 mg. The daily dosage may be given in, e.g. one to three doses.

Preparation of the erosion matrix tablets according to the invention may be obtained by granulation, followed by tableting, enteric coating and optionally film coating of the core tablets obtained. The core can for example be made by conventional wet granulation or continuous granulation such as extrusion followed by compaction of the granules into tablets. The core may then be coated using an appropriate technology, preferably by air suspension.

An aspect of the invention is a method for preparing the formulation according to the invention, comprising the steps of:
a) Dissolving (or suspending) either one or both of a fumaric acid ester and optionally a rate-controlling agent in the form of a polymeric matrix material in water to obtain an aqueous suspension thereof;
b) Spraying said aqueous suspension on granules of a fumaric acid ester and/or a binder for a period of time sufficient to obtain a uniform coating thereon;
c) Drying the granules obtained;
d) Optionally sieving or milling said granules;
e) Blending of any pharmaceutically acceptable excipients and additives in a manner known per se to obtain a tablet formulation;
f) Enteric coating and optionally film coating of said tablet formulation in a manner known per se;
wherein any of or all of the above steps are performed at a temperature to allow a product temperature not exceeding 45 °C.

In an embodiment of the invention any of or all of the above steps are performed at a temperature to allow a product temperature not exceeding 40 °C, such as not exceeding 35 °C, such as not exceeding 30 °C. Thus it has surprisingly been shown that the preparation of the formulation according to the invention may be obtained by the use of solely water as solvent, thus obviating the need for any organic solvents. Furthermore all process steps may be carried out at a rather low temperature. Thereby any sublimation of the active pharmaceutical ingredient is minimised or reduced and an energy-efficient process is obtained, mitigating loss of API, thus reducing cost as well as improving environmental and workers' safety.

In the present context particle size is measured by conventional sieve analysis known to the person skilled in the art.

It is preferred that the mean particle size of the active pharmaceutical ingredient (the dimethyl fumarate) is reduced, e.g. by sieving or milling, such that at least 50% of the particles have a particle size of less than 800 µm, such as less than 600 µm, such as less than 500 µm, such as less than 400 µm, such as less than 300 µm, such as less than 200 µm prior to step a) above.

In another preferred embodiment, the mean particle size of the active pharmaceutical ingredient (the dimethyl fumarate) is reduced, e.g. by sieving or milling, such that at least 80% of the particles have a particle size of less than 800 µm, such as less than 600 µm, such as less than 500 µm, such as less than 400 µm, such as less than 200 µm, prior to step a) above.

According to a preferred embodiment, the mean particle size of the crystalline active pharmaceutical ingredient dimethyl fumarate is reduced, e.g. by sieving or milling, such that at least 90% of the particles have a particle size of less than 800 µm, such as less than 600 µm, such as less than 500 µm, such as less than 400 µm, such as less than 200 µm, prior to step a) above.

In an embodiment of the invention the mean particle size of the crystalline active pharmaceutical ingredient dimethyl fumarate may be reduced, e.g. by sieving or milling, wherein said sieving or milling is performed producing a minimum amount of heat. Thereby any sublimation of the active pharmaceutical ingredient is minimised or reduced and an energy-efficient process is obtained, mitigating loss of API, thus reducing cost as well as improving environmental and workers' safety. The sieving or milling may take place as a single sieving or milling step or may optionally be repeated several times to obtain the required particle distribution.

In one embodiment of the invention, the sieving or milling takes place as a two-step process.

In one embodiment of the invention, where the sieving or milling is performed as several steps an agent for reducing agglomeration is added in between the steps.

In one aspect, a lower amount of rate-controlling agent enables manufacture of a tablet with a high drug load such as at least 35%, 40%, 45 %, 50 %, 55%, or 60% dimethyl fumarate based on the total tablet weight.

In an embodiment of the invention step b) is performed in a fluid bed granulator.

Another aspect of the invention is a method for preparing the formulation according to the invention, comprising the steps of:
a) Dissolving (or suspending) a rate-controlling agent in the form of a polymeric matrix material in water to obtain an aqueous suspension thereof;
b) Spraying said aqueous suspension on granules of a fumaric acid ester for a period of time sufficient to obtain a uniform coating thereon;
c) Drying the granules obtained;
d) Optionally sieving or milling said granules;
e) Blending of any pharmaceutically acceptable excipients and additives in a manner known per se to obtain a tablet formulation;
f) Enteric coating and optionally film coating of said tablet formulation in a manner known per se;
wherein any of or all of the above steps are performed at a temperature to allow a product temperature not exceeding 45 °C. In an embodiment of the invention any of or all of the above steps are performed at a temperature to allow a product temperature not exceeding 40 °C, such as not exceeding 35 °C, such as not exceeding 30 °C. Thereby any sublimation of the active pharmaceutical ingredient is minimised or reduced and an energy-efficient process is obtained, mitigating loss of API, thus reducing cost as well as improving environmental and workers' safety.

In an embodiment of the invention step b) is performed in a fluid bed granulator.

Another embodiment of the invention is a method for preparing the formulation according to the invention, comprising the steps of:
a) sieving and /or milling crystals of fumaric acid ester;
b) Blending of said crystals of fumaric acid ester, optionally a rate- controlling agent in the form of a polymeric matrix material, and any pharmaceutically acceptable excipients and additives by direct compression to obtain a tablet formulation;
c) Enteric coating and optionally film coating of said tablet formulation in a manner known per se;
wherein any of or all of the above steps are performed at a temperature to allow a product temperature not exceeding 45 °C. In an embodiment of the invention any of or all of the above steps are performed at a temperature to allow a product temperature not exceeding 40 °C, such as not exceeding 35 °C, such as not exceeding 30 °C. Thereby any sublimation of the active pharmaceutical ingredient is minimised or reduced and an energy-efficient process is obtained, mitigating loss of API, thus reducing cost as well as improving environmental and workers' safety.

Another embodiment of the invention is a method for preparing the formulation according to the invention, comprising the steps of:
a) Blending of crystals of fumaric acid ester, optionally a rate- controlling agent in the form of a polymeric matrix material, and any pharmaceutically acceptable excipients and additives;
b) milling the blended mix, add more additives and obtain by direct compression the tablet formulation;
c) Enteric coating and optionally film coating of said tablet formulation in a manner known per se;
wherein any of or all of the above steps are performed at a temperature to allow a product temperature not exceeding 45 °C. In an embodiment of the invention any of or all of the above steps are performed at a temperature to allow a product temperature not exceeding 40 °C, such as not exceeding 35 °C, such as not exceeding 30 °C. Thereby any sublimation of the active pharmaceutical ingredient is minimised or reduced and an energy-efficient process is obtained, mitigating loss of API, thus reducing cost as well as improving environmental and workers' safety.

Another embodiment of the invention is a method for preparing the formulation according to the invention, comprising the steps of:
a) Optionally sieving or milling crystals of fumaric acid ester;
b) Blending said crystals of fumaric acid ester with any pharmaceutically acceptable excipients and optionally a rate-controlling agent in the form of a polymeric matrix material in a manner known per se to obtain a tablet formulation;
c) Roller compaction of this blend and sieving/milling thereof in order to obtain granules;
d) Admixing of any further pharmaceutically acceptable excipients to the granules to obtain a final mix ready for tabletting;
e) Compression to tablets;
f) Enteric coating and optionally film coating of said tablets.

In an embodiment of the invention the fumaric acid ester is preblended with one or more pharmaceutically acceptable excipients before step a) above.

The stability of the formulations according to the invention may be determined by measuring the initial *in vitro* dissolution profile of the tablets and the *in vitro* dissolution profile after different periods of storage and comparing the *in vitro* dissolution profiles obtained. In an embodiment of the invention the tablets are stable for at least 6 months, such as at least 9 months, such as at least 12 months, such as at least 18 months, such as at least 24 months, such as 36 months.

The stability of the formulations according to the invention may also be determined by standardized methods for measuring any changes in for example assay, colour or degradation products.

In an embodiment of the invention, stability of a formulation can be defined by objective criteria, such as e.g. a certain maximum change of the amount of API released at a predetermined time point during a standardized *in vitro* dissolution test, when comparing the initial testing time point to testing at a later point in time. In an embodiment of the invention, the amount of the API released from the formulation stored under ICH conditions (such as degrees C/60% RH, such as 30 degrees C/65% RH, such as 40 degrees C/75% RH) for a certain period of time (such as at least 1 month, such as at least 3 months, such as at least 6 months, such as at least 9 months, such as at least 12 months, such as at least 18 months, such as at least 24 months, such as at least 36 months) c.f. the initial time point (time = 0, set down of stability testing) - when subjected to an *in vitro* dissolution test employing 0.1 N hydrochloric acid as dissolution medium during the first 2 hours of the test and then 0.05 M phosphate buffer pH 6.8 as dissolution medium - is as follows:
1 hour after start of the test, a difference of less than 10 percentage points, such as less than 9 percentage points, such as less than 8 percentage points, such as less than 6 percentage points, such as less than 4 percentage points, such as less than 2 percentage points, such as less than 1 percentage point in the amount of the active pharmaceutical ingredient released from the formulation is observed, and/or
2 hours after start of the test, a difference of less than 10 percentage points, such as less than 9 percentage points, such as less than 8 percentage points, such as less than 6 percentage points, such as less than 4 percentage points, such as less than 2 percentage points, such as less than 1 percentage point in the amount of the active pharmaceutical ingredient released from the formulation is observed, and/or
3 hours after start of the test, a difference of less than 10 percentage points, such as less than 9 percentage points, such as less than 8 percentage points, such as less than 6 percentage points, such as less than 4 percentage points, such as less than 2 percentage points, such as less than 1 percentage point in the amount of the active pharmaceutical ingredient released from the formulation is observed, and/or
4 hours after start of the test, a difference of less than 10 percentage points, such as less than 9 percentage points, such as less than 8 percentage points, such as less than 6 percentage points, such as less than 4 percentage points, such as less than 2 percentage points, such as less than 1 percentage point in the amount of the active pharmaceutical ingredient released from the formulation is observed, and/or
5 hours after start of the test, a difference of less than 10 percentage points, such as less than 9 percentage points, such as less than 8 percentage points, such as less than 6 percentage points, such as less than 4 percentage points, such as less than 2 percentage points, such as less than 1 percentage point in the amount of the active pharmaceutical ingredient released from the formulation is observed.

The pharmaceutical formulation according to the present invention is for use for the treatment of psoriasis, including mild to moderate, moderate to severe, or severe plaque psoriasis.

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims. Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges and are encompassed within the invention, subject to any specifically excluded limit in the stated range. Where the stated range includes one or both of the limits, ranges excluding either or both of those included limits are also included in the invention. Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are described. All publications mentioned herein are incorporated herein by reference to disclose and describe the methods and/or materials in connection with which the publications are cited. It must be noted that as used herein and in the appended claims, the singular forms "a", "an", and "the" include plural referents unless the context clearly dictates otherwise.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

### EXAMPLES

During the carrying out of all the following steps in the examples necessary precautions are taken (protective clothing with external air supply, double gloves, arm covers, breathing mask, etc and/or contained equipment have to be used.).

### Example 1

1.2 kg dimethyl fumarate was sieved through a 700 µm sieve and placed in the basket of a fluid bed granulator. 70.6 g polymer hydroxypropyl cellulose HPC-SL was dissolved by stirring in 2753 g purified water and sprayed on the DMF over 2.5 to 3 hours. The granules were dried for 3 minutes at 29°C. Several batches were blended and sieved through a 800 µm sieve.

1730.7 g of the dried and additional through 500 µm sieved granules were blended with 25 781.3 g granulated lactose (Tablettose ® 100), 66.7 g HPC-SL and a pre-blend of Aerosil® and Tablettose® with a barrel blender at 20 rpm over 15 minutes. The pre-blend was prepared in a polyethylene bag of 4 g colloidal silicic acid (Aerosil®) and 390.6 g Tablettose® and sieved through 500 µm. Finally, 26.7 g magnesium stearate was added. The final blend was pressed into biconvex tablets with a diameter of 8 mm and a weight of 225 mg.

### Example 2

Film and enteric coating of core tablets according to example 1.

### Film coating

For film coating of 800 g core tablets a 15% suspension of Opadry was prepared by adding 18 g Opadry to 102 g purified water. App. 66% of this suspension was sprayed onto the core tablets over 20 minutes in a fluid bed chamber. The product temperature never exceeded 40°C. The coating process was followed by a drying period of 9 minutes at 30 °C. The achieved coating was less than 0.7% weight increase compared to the core tablet weight.

### Enteric coating

1 kg gastric acid-resistant coating fluid was prepared by heating 350 ml purified water to 70 - 80°C, adding 9.5 g triethyl citrate, 1.9 g glyceryl monostearate (Cutina GMS V), 0.7 g Tween 80 and stirring with the UltraTurrax for 10 minutes to achieve a homogenous mixture. 427.8 g purified water was added and the mixture was stirred with a propeller stirrer until the emulsion had reached room temperature. This emulsion was then added slowly to 210 g of a Eudragit ® L30 D 55 dispersion. Approximately 66% of the resulting gastric acid-resistant coating fluid was sprayed on 780 g film-coated tablets in a fluid bed chamber. The real applied coating level was app. 2.2% weight increase compared to the core tablet due to a high loss during the process.

### Example 3

1.2 kg dimethyl fumarate was sieved through a 700 µm sieve and placed in the basket of a fluid bed granulator. 70.6 g hydroxypropyl cellulose HPC-SL was dissolved by stirring in 2753 g purified water and sprayed on the DMF over 2.5 to 3 hours. The granules were dried for 3 minutes at 29°C and sieved through a 500 µm sieve.

964 g of the dried, sieved granules were blended with 565.5 g granulated lactose (Tablettose® 100), 37.4 g HPC-SL and a pre-blend of Aerosil® and Tablettose® with a barrel blender at 20 rpm over 15 minutes. The pre-blend was prepared in a polyethylene bag of 2.3 g colloidal silicic acid (Aerosil®) and 282.7 g Tablettose® and sieved through 500 µm as well. Finally, 14.9 g magnesium stearate was added. The final blend was pressed into 30 biconvex tablets with a diameter of 8 mm and a weight of 250 mg.

### Example 4

Film and enteric coating of core tablets according to example 3.

### Film coating

For film coating of 800 g core tablets a 15% suspension of Opadry is prepared and applied as disclosed in example 2.

### Enteric coating

1 kg gastric acid-resistant coating fluid was prepared by heating 350 ml purified water to 70 - 80°C, adding 9.5 g triethyl citrate, 1.9 g glyceryl monostearate (Cutina GMS V), 0.7 g Tween 80 and stirring with the UltraTurrax for 10 minutes to achieve a homogenous mixture. 427.8 10 g purified water was added and the mixture was stirred with a propeller stirrer until the emulsion had reached room temperature. This emulsion was then added slowly to 210 g of a Eudragit ® L30 D 55 dispersion. Approximately 66% of the resulting gastric acid-resistant coating fluid was sprayed on 780 g film coated tablets in a fluid bed chamber at a temperature of 30 °C over app. 2.5 hours. A drying period at 30°C for 30 minutes and a 15 curing period at 35°C for additional 30 minutes followed. The real applied coating level was app. 2.2% weight increase compared to the core tablet due to a high loss during the process.

### Example 5

1.2 kg dimethyl fumarate was sieved through a 700 µm sieve and placed in the basket of a fluid bed granulator. 70.6 g hydroxypropyl cellulose HPC-SL was dissolved by stirring in 2753 g purified water and sprayed on the DMF over 2.5 to 3 hours. The granules were dried for 3 minutes at 29°C. Several batches were blended and sieved through 800 µm.

1416 g of the dried and additional through 500 µm sieved granules were blended with 1002.9 g granulated lactose (Tablettose® 100), 54.6 g HPC-SL and a pre-blend of Aerosil® and Tablettose® with a barrel blender at 20 rpm over 15 minutes. The pre-blend was prepared in a polyethylene bag of 3.3 g colloidal silicic acid (Aerosil®) and 501.4 g Tablettose® and sieved through 500 µm. Finally, 21.8 g magnesium stearate was added. The final blend was pressed into biconvex tablets with a diameter of 8 mm and a weight of 275 mg.

### Example 6

Film and enteric coating of core tablets according to example 5.

### Film coating

For film coating of 800 g core tablets a 15% suspension of Opadry was prepared and applied as disclosed in example 2.

### Enteric coating

1 kg gastric acid-resistant coating fluid was prepared by heating 350 ml purified water to 70 - 80°C, adding 9.5 g triethyl citrate, 1.9 g glyceryl monostearate (Cutina GMS V), 0.7 g Tween 80 and stirring with the UltraTurrax for 10 minutes to achieve a homogenous mixture. 427.8 g purified water was added and the mixture was stirred with a propeller stirrer until the emulsion had reached room temperature. This emulsion was then added slowly to 210 g of a Eudragit® L30 D 55 dispersion. Approximately 66% of the resulting gastric acid-resistant coating fluid was sprayed on 780 g film coated tablets in a fluid bed chamber at a temperature of 30 °C over app. 2.5 hours. A drying period at 30°C for 30 minutes and a curing period at 35°C for additional 30 minutes followed. The real applied coating level was app. 2.2% weight increase compared to the core tablet due to a high loss during the process.

### Example 7

Film coating of core tablets according to example 2.

### Film coating

For film coating of 800 g core tablets a 15% suspension of Opadry was prepared by adding 36 g Opadry to 204 g purified water. App. 66% of this suspension was sprayed onto the core tablets over 35 minutes in a fluid bed chamber. The product temperature never exceeded 40°C. The coating process was followed by a drying period of 16 minutes at 30 °C.. The achieved coating was less than 2% weight increase compared to the core tablet weight.

### Example 8

18 g of pure DMF (particle size 250-500 µm) were blended with 6.3 g HPC-SL, 9.1 g spray dried lactose (FlowLac ® 100) and 0.045 g Aerosil. Finally, 0.3 g magnesium stearate was added and blended. The final blend was pressed into biconvex tablets with a diameter of 8 mm and a weight of 225 mg.

### Example 9

The study was a single center study, following an open-label, randomized, crossover design to investigate the plasma concentrations, pharmacokinetics, safety and tolerability of pharmaceutical formulations according to the invention c.f. the marketed formulation Fumaderm® as reference. The tablets were administered as a single oral dose of 240 mg (2 tablets containing 120 mg each) in each treatment period according to randomization to 20 healthy, male Caucasian subjects. The study was divided into four treatment periods (Treatment Period 1, 2, 3 and 4), which were separated by a wash-out phase of at least 7 days. Subjects were screened for eligibility at least 21 to 2 days before first administration including: check of inclusion / exclusion criteria; demographic data (including age, body height, body weight, body mass index (BMI), and ethnic origin); physical examination; complete medical history; 12-lead electrocardiogram (ECG); vital signs (blood pressure (BP), pulse rate (PR), and body temperature (BT)); clinical laboratory parameters (hematology, 20 serum biochemistry, and urinalysis); documentation of concomitant illness and medication.

At each of the four treatment periods, subjects came to the Study Site in the evening of Day 1 and remained there until the 24-hour blood sample for PK analysis was drawn and all safety measurements were performed (=morning of Day 2).

The subjects fasted overnight. A single oral dose (of two tablets) of one of the formulations according to the invention (Examples 2, 4 or 6), or two enteric-coated tablets of the reference medication Fumaderm® each containing 120 mg dimethyl fumarate (total dose 240 mg dimethyl fumarate) were administered on Day 1 (according to randomization). Administration was done to subjects who were in fasting condition together with 240 ml tap water. Between each administration, a wash-out interval of at least 7 days was maintained.

The following assessments/measurements were performed:
Blood sampling was performed for the determination of plasma concentrations and PK-parameters prior to, and at pre-scheduled times post dosing.

Adverse events were documented in detail throughout the study.

Urine was collected prior to and at pre-scheduled times post dosing.

A follow-up examination was performed at least 7 days after the last administration (Treatment Period 4), including: physical examination; vital signs (BP, PR, and BT); body weight; 12-lead ECG; clinical laboratory parameters (haematology, serum biochemistry, and urinalysis); documentation of concomitant medication and adverse events.

### Example 10

### Preparation of core tablets

Dimethyl fumarate was sieved through a hand screen of 500 µm.

29.3 g of sieved dimethyl fumarate, 2.93 g of HPC-SL, 22.17 g of granulated lactose (Tablettose® 100), 0.07 g of Aerosil® as well as 0.49 g of magnesium stearate were blended for 10 minutes. The blend was pressed into biconvex tablets with a diameter of 8 mm and a weight of 225 mg.

### Example 11

### Preparation of core tablets

Dimethyl fumarate was sieved through a hand screen of 500 µm.

500 g of sieved dimethyl fumarate, 48 g of HPC-SL, 447 g of spray dried lactose (FlowLac® 100) and 1.2 g Aerosil® were blended with a barrel blender for 15 minutes at 20 rpm.
Finally, 4 g of magnesium stearate was added and the mixture blended again for 10 min at 20 rpm. The blend was pressed into biconvex tablets with a diameter of 8 mm and a weight of 250 mg.

### Enteric coating

A gastric acid-resistant coating fluid was prepared by heating 247 g of purified water to 70 - 80°C, then 9 g of triethyl citrate, 1.8 g of glyceryl monostearate (Cutina GMS V), and 0.72 g of Tween 80 was added and stirred with the UltraTurrax for 10 minutes to achieve a homogenous mixture. 495 g of purified water was added and the mixture was stirred with a propeller stirrer until the emulsion had reached room temperature. This emulsion was then added slowly to 200 g of Eudragit L30 D 55 dispersion. The resulting gastric acid-resistant coating fluid was sprayed on the core tablets directly in a perforated drum coater. The amount of solution sprayed onto the tablets was 2.5% solids w/w resulting in a weight increase of the coated tablets compared to core tablets of 1.8%.

### Example 12

### Preparation of core tablets

Dimethyl fumarate was sieved through a hand screen of 500 µm.

500 g sieved dimethyl fumarate, 48 g of HPC-SL and 447 g of granulated lactose (Tablettose® 100) and 1.2 g Aerosil® were blended with a barrel blender for 15 minutes at 20 rpm. Finally, 4 g of magnesium stearate was added and the mixture blended again for 10 min at 20 rpm. The blend was pressed into biconvex tablets with a diameter of 8 mm and a weight of 250 mg.

### Enteric coating

A gastric acid-resistant coating fluid was prepared by heating 99 g of purified water to 70 - 80°C, then 10.1 g of triethyl citrate, 2.0 g of glyceryl monostearate (Cutina GMS V), and 0.8 g of Tween 80 was added and stirred with the UltraTurrax for 10 minutes to achieve a homogenous mixture. 198 g of purified water was added and the mixture was stirred with a propeller stirrer until the emulsion had reached room temperature. This emulsion was then added slowly to 224 g of Eudragit L30 D 55 dispersion. The resulting gastric acid-resistant coating fluid was sprayed on the core tablets directly in a perforated drum coater. The solution was sprayed to a weight increase of the core tablets of 3%.

### Example 13a

### Preparation of core tablets

Dimethyl fumarate was milled through 1143 um and 610 um screens.

500 g sieved dimethyl fumarate, 48 g of HPC-SL and 447 g of granulated lactose (Tablettose® 100) and 1.2 g of Aerosil® were blended with a barrel blender for 15 minutes at 20 rpm. Finally, 4 g of magnesium stearate was added and the mixture blended again for 10 min at 20 rpm. The blend was pressed into biconvex tablets with a diameter of 8 mm and a weight of 250 mg.

### Enteric coating

A gastric acid-resistant coating fluid was prepared by heating 247 g of purified water to 70 - 80°C, then 9 g of triethyl citrate, 1.8 g of glyceryl monostearate (Cutina GMS V), and 0.72 g of Tween 80 was added and stirred with the UltraTurrax for 10 minutes to achieve a homogenous mixture. 495 g of purified water was added and the mixture was stirred with a propeller stirrer until the emulsion had reached room temperature. This emulsion was then added slowly to 200 g of Eudragit L30 D 55 dispersion. The resulting gastric acid-resistant coating fluid was sprayed on the core tablets directly in a perforated drum coater. The amount of solids sprayed onto the tablets was 2.5% solids w/w resulting in a weight increase of the coated tablets compared to core tablets of 1.5%.

### Example 13b

Preparation of core tablets was performed as described in Example 13a.

### Enteric coating

A gastric acid-resistant coating fluid was prepared by heating 247 g of purified water to 70 - 80°C, then 9 g of triethyl citrate, 1.8 g of glyceryl monostearate (Cutina GMS V), and 0.72 g of Tween 80 was added and stirred with the UltraTurrax for 10 minutes to achieve a homogenous mixture. 495 g of purified water was added and the mixture was stirred with a propeller stirrer until the emulsion had reached room temperature. This emulsion was then added slowly to 200 g of Eudragit L30 D 55 dispersion. The resulting gastric acid-resistant coating fluid was sprayed on the core tablets directly in a perforated drum coater. The amount of solids sprayed onto the tablets was 3.5% solids w/w resulting in a weight increase of the coated tablets compared to core tablets of 2%.

### Example 14

### Preparation of core tablets

2500 g of dimethyl fumarate was milled through 1575 µm and 813 µm screens. Before the second milling step 6 g of Aerosil® was added. The achieved particle size distribution was approx. 3% > 500 µm, approx. 65% > 250 µm and approx. 6% < 100 µm. The mean particle size was 290 µm.

The milled material was blended further with 240 g of HPC-SL and 2714 g of granulated lactose (Tablettose® 100) with a barrel blender for 15 minutes at 20 rpm. Finally, 20 g of magnesium stearate was added and the mixture blended again for 10 min at 20 rpm. The blend was pressed into biconvex tablets with a diameter of 8 mm and a weight of 275 mg. The core tablets may be enteric coated as described in example 16a or b.

### Example 15

### Preparation of core tablets

2500 g of dimethyl fumarate was milled through 1575 µm and 813 µm screens. Before the second milling step 6 g of Aerosil® was added. The achieved particle size distribution was approx. 3% > 500 µm, approx. 50% > 250 µm and approx. 10% < 100 µm. The mean particle size was 250 µm.

The milled material was blended further with 240 g of HPC-SL and 2714 g of granulated lactose (Tablettose® 100) with a barrel blender for 15 minutes at 20 rpm. Finally, 20 g of magnesium stearate was added and the mixture blended again for 10 min at 20 rpm. The blend was pressed into biconvex tablets with a diameter of 8 mm and a weight of 275 mg. The core tablets were optionally enteric coated as described in example 16b.

### Example 16a

### Enteric coating

A gastric acid-resistant coating fluid was prepared by heating 1193 g of purified water to 70 - 80°C, then 45 g of triethyl citrate, 13.5 g of glyceryl monostearate (Cutina GMS V), and 5.4 g of Tween 80 was added and stirred with the UltraTurrax for 10 minutes to achieve a homogenous mixture. 2385 g of purified water was added and the mixture was stirred with a propeller stirrer until the emulsion had reached room temperature. This emulsion was then added slowly to 1500 g of Eudragit L30 D 55 dispersion. The resulting gastric acid-resistant coating fluid was sprayed on the core tablets directly in a perforated drum coater. The amount of solids sprayed onto the tablets was 3.0 % w/w resulting in a weight increase of the coated tablets compared to core tablets of 2.5%.

### Example 16b

### Enteric coating

A gastric acid-resistant coating fluid was prepared by heating 1193 g of purified water to 70 - 80°C, then 45 g of triethyl citrate, 13.5 g of glyceryl monostearate (Cutina GMS V), and 5.4 g of Tween 80 was added and stirred with the UltraTurrax for 10 minutes to achieve a homogenous mixture. 2385 g of purified water was added and the mixture was stirred with a propeller stirrer until the emulsion had reached room temperature. This emulsion was then added slowly to 1500 g of Eudragit L30 D 55 dispersion. The resulting gastric acid-resistant coating fluid was sprayed on the core tablets directly in a perforated drum coater. The amount of solids sprayed onto the tablets was 3.5% resulting in a weight increase of the coated tablets compared to core tablets of 3%.

### Example 17

### Preparation of core tablets

2500 g of dimethyl fumarate was milled through 1575 µm and 813 µm screens. Before the second milling step 6 g Aerosil® was added. The achieved particle size distribution was 3% > 500 µm, 63% > 250 µm and 6% < 100 µm. The mean particle size was 290 µm.

The milled material was blended further with 240 g of HPC-SL and 2234 g of granulated lactose (Tablettose® 100) with a barrel blender for 15 minutes at 20 rpm. Finally, 20 g of magnesium stearate was added and the mixture blended again for 10 min at 20 rpm. The blend was pressed into biconvex tablets with a diameter of 8 mm and a weight of 250 mg. The core tablets may be enteric coated as described in example 16a or b.

### Example 18

### Preparation of core tablets

2500 g of dimethyl fumarate is milled through 1575 um and 813 µm screens. Before the second milling step 6 g of Aerosil® is added.

The milled material is blended further with 240 g of HPC-SL and 1714 g of granulated lactose (Tablettose® 100) with a barrel blender for 15 minutes at 20 rpm. Finally, 20 g of magnesium stearate is added and the mixture blended again for 10 min at 20 rpm. The blend is pressed into biconvex tablets with a diameter of 8 mm and a weight of 225 mg. The core tablets may be enteric coated as described in example 16a or b.

### Example 19

2.500 g of DMF is milled through 1575 µm and 813 µm screens. 240 g of HPC-SL, 2.734 g of Tablettose 100 and 6 g of Aerosil is added and blended with the DMF. The blend is roller compacted and passed through a 1 mm screen to obtain granules. 20 g of magnesium stearate is admixed to obtain a final mix ready for tabletting. Said mix is compressed to tablets having a tablet weight of 275 mg. The core tablets may be enteric coated as described in example 16a or b.

### Example 20

2.500g of DMF is blended with 6 g of Aerosil and subsequently milled through 1575 µm and 813 µm screens. 240 g of HPC-SL and 2.734 g of Tablettose 100 is added and blended with the DMF and Aerosil. The blend is roller compacted and passed through a 1 mm screen to obtain granules. 20 g of magnesium stearate is admixed to obtain a final mix ready for tabletting. Said mix is compressed to tablets having a tablet weight of 275 mg. The core tablets may be enteric coated as described in example 16a or b.

### Example 21

A study as the one disclosed in example 25 was performed on tablets as disclosed in examples 18 and 22 and compared with corresponding data for the prior art formulation Fumaderm®. The results of the study are shown in Table I and Table II below.

**Table I**

| Coefficients of variation in % (CV). | | | |
|---|---|---|---|
| | Example 2 | Example 6 | Fumaderm® |
| AUC | 22 % | 18 % | 38 % |
| Cmax | 34 % | 26% | 49 % |

**Table II**

| Summary Table: Percentage of subjects with adverse effects/side effects after administration of formulation according to examples 2 and 6, respectively, compared to administration of Fumaderm® | | |
|---|---|---|
| Adverse effect/side effect | After administration of formulation acc. To ex. 2 c.f. after administration of Fumaderm® | After administration of formulation acc. To ex. 6 c.f. after administration of Fumaderm® |
| Flushing | 35 % | 65 % |
| GI related adverse effects | 50 % | 73 % |
| Any adverse effect | 50 % | 77 % |

The above results of the clinical trial shows (Table II) that the tested formulations have a markedly reduced frequency of adverse effects combined with a lower variability (cf. Table I) compared to Fumaderm®. This example thus shows that the erosion matrix tablets have a large reduction in variability in AUC and Cₘₐₓ vis-à-vis the prior art Fumaderm® formulation.

### Example 22

### Preparation of core tablets

13.4 kg of dimethyl fumarate was milled through 1575 µm and 813 µm screens. Before the second milling step 32 g of Aerosil® was added. The achieved particle size distribution was app. 3% > 500 µm, approx. 52% > 250 µm and approx. 6% < 100 µm. The mean particle size was 255 µm.

12.8 kg of the milled material was blended further with 1.2 kg of HPC-SL and 13.9 kg of granulated lactose (Tablettose® 100). Finally, 0.1 kg of magnesium stearate was added and the mixture blended again. The blend was pressed into biconvex tablets with a diameter of 8 mm and a weight of 275 mg. The core tablets were enteric coated as described below.

### Enteric coating

A gastric acid-resistant coating fluid was prepared by heating 7,2 kg of purified water to 70 - 80°C, then 138 g of triethyl citrate, 41 g of glyceryl monostearate (Cutina GMS V), and 17 g of Tween 80 was added and stirred with the UltraTurrax for 10 minutes to achieve a homogenous mixture. 8,9 kg of purified water was added and the mixture was stirred with a propeller stirrer until the emulsion had reached room temperature. This emulsion was then added slowly to 4.58 kg of Eudragit® L30 D 55 dispersion. The resulting gastric acid-resistant coating fluid was sprayed on the core tablets directly in a perforated drum coater. After that the tables were cured over 2 hours at 40°C.The amount of solids sprayed onto the tablets corresponded to a 2.75% weight increase resulting in a real weight increase of the coated tablets compared to core tablets of app. 2.2%. Based on the shape of the tablet this corresponded to an enteric coat of 2.6 mg/cm².

### Example 23

### Preparation of core tablets

1136 g of dimethyl fumarate was blended with 2.73 g of Aerosil®, 109 g of HPC-SL and 1242.7 g of granulated lactose (Tablettose® 100). This blend was after that milled to a 613 µm screen. Finally, 9.1 g of magnesium stearate was added and the mixture blended again. The blend was pressed into biconvex tablets with a diameter of 8 mm and a weight of 275 mg. The core tablets were optionally enteric coated based on the description of the example before.

### Example 24

### Preparation of core tablets

14.7 kg of dimethyl fumarate was blended with 36 g of Aerosil® and de-agglomerated by milling through a 613 µm screens - app. 3% > 500 µm, approx. 20% > 250 µm and approx. 25% < 100 µm. The mean particle size was 165 µm.

13.7 kg of the milled material was blended further with 1.3 kg of HPC-SL and 14.9 kg of granulated lactose (Tablettose® 100). Finally, 0.11 kg of magnesium stearate was added and the mixture blended again. The blend was pressed into biconvex tablets with a diameter of 8 mm and a weight of 275 mg. The core tablets were enteric coated as described below.

### Enteric coating

A gastric acid-resistant coating fluid was prepared by heating 8.7 kg of purified water to 70 - 80°C, then 166 g of triethyl citrate, 49 g of glyceryl monostearate (Cutina GMS V), and 20 g of Tween 80 was added and stirred with the UltraTurrax for 10 minutes to achieve a homogenous mixture. 10.7 kg of purified water was added and the mixture was stirred with a propeller stirrer until the emulsion had reached room temperature. This emulsion was then added slowly to 5.5 kg of Eudragit® L30 D 55 dispersion. The resulting gastric acid-resistant coating fluid was sprayed on the core tablets directly in a perforated drum coater. After that the tables were cured over 2 hours at 40°C. The amount of solids sprayed onto the tablets corresponded to a 2.75% weight increase resulting in a real weight increase of the coated tablets compared to core tablets of app. 2.4%. Based on the shape of the tablet this corresponded to an enteric coat of 2.9 mg/cm².

### Example 25

### Preparation of core tablets

13.3 kg of dimethyl fumarate was blended with 32 g of Aerosil® and de-agglomerated by milling through a 613 µm screens - app. 3% > 500 µm, approx. 20% > 250 µm and approx. 25% < 100 µm. The mean particle size was 165 µm.

12.53 kg of the milled material was blended further with 1.2 kg of HPC-SL and 16.17 kg of granulated lactose (Tablettose® 100). Finally, 0.10 kg of magnesium stearate was added and the mixture blended again. The blend was pressed into biconvex tablets with a diameter of 11.5 mm and a weight of 600 mg. The core tablets were enteric coated as described below.

### Enteric coating

A gastric acid-resistant coating fluid was prepared by heating 8.7 kg of purified water to 70 - 80°C, then 166 g of triethyl citrate, 49 g of glyceryl monostearate (Cutina GMS V), and 20 g of Tween 80 was added and stirred with the UltraTurrax for 10 minutes to achieve a homogenous mixture. 10.7 kg of purified water was added and the mixture was stirred with a propeller stirrer until the emulsion had reached room temperature. This emulsion was then added slowly to 5.5 kg of Eudragit® L30 D 55 dispersion. The resulting gastric acid-resistant coating fluid was sprayed on the core tablets directly in a perforated drum coater. After that the tables were cured over 2 hours at 40°C. The amount of solids sprayed onto the tablets corresponded to a 2.3% weight increase resulting in a real weight increase of the coated tablets compared to core tablets of app. 2%. Based on the shape of the tablet this corresponded to an enteric coat of 2.8 mg/cm².

### Example 26

A unit dosage form consisting essentially of 375 mg ± 5%, 250 mg ± 5%, 187.5 mg ± 5%, or 125 mg ± 5% dimethyl fumarate formulated as an enterically coated erosion matrix tablet is prepared using the methods described in, for example, Examples 4, 6, 7, 10, 12, 13a, 13b, 16a, 16b, 20, and 24. In one embodiment, the 375 mg dose of dimethyl fumarate is administered once daily. In another embodiment, the 187.5% mg dose is administered twice daily. And in yet another embodiment, the 125 mg dose is administered three times daily. Such low dosing regimens are especially advantageous in reducing side effects associated with dimethyl fumarate therapies.

In view of the foregoing, it will be appreciated that the invention described herein inter alia relates to the following items:
1. A pharmaceutical composition for oral use in treating psoriasis, wherein said composition is in the form of an erosion matrix tablet comprising a tablet core and one or more coating(s), wherein the tablet core comprises
   i) 10 to 80 % by weight dimethyl fumarate as an active substance, and
   ii) 1 to 50 % by weight of one or more rate-controlling agents,
   wherein at least one of the one or more coating(s) is an enteric coating applied at a level of 1.5 to 3.5 % by weight of the core, and
   wherein the dose of dimethyl fumarate to be administered is 375 mg ± 5% per day.
2. The pharmaceutical composition according to item 1, wherein the dose to be administered is 375 mg per day.
3. The pharmaceutical composition according to item 1 or 2, wherein the tablet core comprises
   i) 30 to 60 % by weight of dimethyl fumarate; and
   ii) 3 to 40 % by weight of one or more rate-controlling agents.
4. The pharmaceutical composition according to item 3, wherein the rate-controlling agent is a water-soluble polymer.
5. The pharmaceutical composition according to any one of items 1-4, wherein the rate-controlling agent is a cellulose polymer or a cellulose derivative or a mixture thereof.
6. The pharmaceutical composition according to any one of the previous items , wherein the rate-controlling agent is selected from the group comprising hydroxypropyl cellulose, hydroxypropyl methyl cellulose (HPMC), methyl cellulose, carboxymethyl cellulose and mixtures thereof.
7. The pharmaceutical composition according to item 6, wherein the rate-controlling agent is hydroxypropyl cellulose.
8. The pharmaceutical composition according to any one of the preceding items, wherein the tablet core further comprises a binder.
9. The pharmaceutical composition according to item 8, wherein the binder is lactose.
10. The pharmaceutical composition according to any one of the preceding items, wherein the tablet core comprises:
   i) 35 - 55 % by weight of dimethyl fumarate;
   ii) 3 - 12 % by weight of hydroxypropyl cellulose; and
   iii) 40 - 60 % by weight of lactose.
11. The pharmaceutical composition according to any one of the preceding items, wherein the tablet core comprises:
   i) 40 - 50 % by weight of dimethyl fumarate;
   ii) 3 - 12 % by weight of hydroxypropyl cellulose; and
   iii) 45 - 55 % by weight of lactose.
12. The pharmaceutical composition according to any one of the preceding items, wherein the tablet core comprises:
   i) 42 - 48 % by weight of dimethyl fumarate;
   ii) 3 - 10 % by weight of hydroxypropyl cellulose; and
   iii) 45 - 52 % by weight of lactose.
13. The pharmaceutical composition according to item 11 or 12, wherein the amount of hydroxypropyl cellulose is 3 - 6 % by weight.
14. The pharmaceutical composition according to any one of the preceding items, wherein the tablet core further comprises 0.15 - 0.7 % by weight of magnesium stearate and, optionally, 0.05 to 0.25 % by weight of silicon dioxide.
15. The pharmaceutical composition according to any one of the preceding items for administration once, twice or three times daily.
16. The pharmaceutical composition according to any one of the preceding items For the treatment of mild to moderate, moderate to severe, or severe plaque psoriasis.

## Claims

1. A pharmaceutical formulation for oral use in treating psoriasis comprising an erosion matrix which comprises:
i) 10 % to 80 % by weight of dimethyl fumarate as an active substance; and
ii) 1 % to 50 % by weight of one or more rate-controlling agents;
wherein the pharmaceutical formulation further comprises lactose; and
wherein the dose of dimethyl fumarate to be administered is 375 mg ± 5% per day.

2. The pharmaceutical formulation for use according to claim 1, wherein the rate-controlling agent is a water-soluble polymer.

3. The pharmaceutical formulation for use according to claim 1 or claim 2, wherein the rate controlling agent is a cellulose polymer or a cellulose derivative or a mixture thereof.

4. The pharmaceutical formulation for use according to any one of claims 1 to 3, wherein the erosion matrix comprises 20 % to 70 % by weight of dimethyl fumarate.

5. The pharmaceutical formulation for use according to any one of claims 1 to 4, wherein the erosion matrix comprises 20 % to 60 % by weight of dimethyl fumarate.

6. The pharmaceutical formulation for use according to any one of claims 1 to 5, wherein the lactose is agglomerated lactose.

7. The pharmaceutical formulation for use according to any one of claims 1 to 5, wherein the lactose is spray-dried lactose.

8. The pharmaceutical formulation for use according to any one of claims 1 to 5, wherein the lactose is abrasive lactose.

9. The pharmaceutical formulation for use according to any one of claims 1 to 8, wherein the pharmaceutical formulation further comprises one or more lubricants.

10. The pharmaceutical formulation for use according to claim 9, wherein the lubricant is magnesium stearate.

11. The pharmaceutical formulation for use according to any one of claims 1 to 10, wherein the pharmaceutical formulation further comprises a flow control agent.

12. The pharmaceutical formulation for use according to claim 11, wherein the flow control agent is silicon dioxide.

13. The pharmaceutical formulation for use according to any one of claims 1 to 12, wherein the pharmaceutical formulation is for administration once, twice or three times daily.

14. The pharmaceutical formulation for use according to any one of claims 1 to 12, wherein the pharmaceutical formulation is for administration once daily.

15. The pharmaceutical formulation for use according to any one of claims 1 to 12, wherein the pharmaceutical formulation is for administration three times daily.
